# EUROPEAN PATENT APPLICATION

(11) **EP 2 031 054 A1**
(43) Date of publication of application: **04.03.2009**
(21) Application number: 08014909.9
(22) Date of filing: 22.08.2008
(51) Int. Cl.: C12N 9/02, C12N 15/62, A61K 38/44

(54) **Fusion protein, gene related to fusion protein, vector, transformants, and anti-inflammatory medicinal composition**

(30) Priority: 31.08.2007 JP 2007225833
(71) Applicant: Nipro Corporation, Kita-ku Osaka-shi, Osaka 531-8510 (JP)
(72) Inventor: Otagiri, Masaki, Kumamoto-shi Kumamoto 861-8038 (JP); Ikuta, Shotaro, Osaka-shi Osaka 531-8510 (JP)
(74) Representative: Leissler-Gerstl, Gabriele

(57) **Abstract**

Fusion protein comprises a thioredoxin and human serum albumin. The thioredoxin comprises a protein comprising the amino acid sequence shown in SEQ. ID. No.1, or the like, the human serum albumin comprises a protein comprising the amino acid sequence shown in SEQ. ID. No. 2, or the like. The fusion protein of the invention allows the activity of thioredoxin to be maintained over a long period of time. It is thus possible to provide a protein preparation that is stable over long periods of time.

## Description

### BACKGROUND OF THE INVENTION

### 1. Field of the Invention

The present invention relates to a novel fusion protein, gene related to the fusion protein, vector, transformants, and anti-inflammatory medicinal composition, and in particular to a fusion protein of human serum albumin and a thioredoxin allowing the activity of thioredoxins to be maintained over a long period of time, as well as a gene related to the fusion protein, a vector, transformants, and an anti-inflammatory medicinal composition.

### 2. Background Information

Thioredoxins are electron transfer proteins with a molecular weight of 10,000 to 13,000, which act as direct electron donors allowing ribonucleotide reductase to reduce ribonucleoside diphosphates to deoxyribonucleoside diphosphates. Thioredoxins also have a pair of functional thiols (SH) and form disulfide bonds with the reduction of ribonucleotides.
These thioredoxins reportedly alleviate ischemic reperfusion injury in a variety of sites in the living body. Diverse types of physiological action such as antioxidant and anti-inflammatory action have also become clear (see JP-2000-103743-A, JP2004-137212-A and JP-2005-29521-A). Thioredoxins therefore hold promise as a useful protein agent.
However, a problem with thioredoxins is their poor retention in blood.

### SUMMARY OF THE INVENTION

An object of the present invention is to provide a novel fusion protein allowing stable thioredoxin activity to be maintained over long periods of time, as well as a gene related to the fusion protein, a vector, transformants, and an anti-inflammatory medicinal composition.

In an effort to utilize thioredoxins as useful protein agents, the inventors first focused on the short half life in blood and conducted extensive research on ways to bring about longer retention in blood in order to ensure that the effects of the protein last longer so as to avoid the complications of multiple short-term dosing. As a result, the inventors discovered that various serum molecules function as carriers in the living body, and that it is possible to use serum albumin characterized by ample stability, biocompatibility, biodegradability, and retention in blood. The present invention was perfected upon the knowledge that the fusion of serum albumin with thioredoxins unexpectedly prolonged the half life in blood and allowed thioredoxin dosing intervals to be extterminaled.

The present invention provides a fusion protein comprising a thioredoxin and human serum albumin.
Also, the present invention provides a gene encoding
(a) a protein comprising the amino acid sequence shown in SEQ. ID. No. 3, or
(b) a protein comprising the amino acid sequence in SEQ. ID. No. 3 with one or more amino acid substitutions, deletions, insertions, and/or additions, that encodes a protein which is functionally equivalent to the protein comprising the amino acid sequence in SEQ. ID. No. 3.

Further, the present invention provides a vector comprising the gene according to the above.

Moreover, the present invention provides a ransformant comprising the vector according to the above.
The present invention provides a fusion protein produced by the transformant according to the above.

Still further, the present invention provides an anti-inflammatory medicinal composition comprising the fusion protein according to the above.

The fusion protein of the invention is such that a thioredoxin is made into a fusion protein with human serum albumin, ensuring more stable thioredoxin activity over a long period of time.
The vector and transformants of the invention allow the fusion protein to be readily produced by recombinant methods.
The anti-inflammatory medicinal composition of the invention can also provide more stable anti-inflammatory action over longer periods of time.

### BRIEF DESCRIPTION OF THE DRAWINGS

Figure 1 is an electropherogram showing the result of Test Example 1.
Figure 2 is a diagram showing the result for Western blotting of the fusion protein of Test Example 2 using anti-human serum albumin antibody and anti-thioredoxin antibody as primary antibody.
Figure 3 is a graph showing the result for ELISA of the fusion protein of Test Example 3.
Figure 4 is a graph showing the thioredoxin activity of the fusion protein of Test Example 4.
Figure 5 is a graph showing the retention of the fusion protein in blood of Test Example 5.

### DETAILED DESCRIPTION OF THE PREFERRED EMBODIMENTS

The fusion protein of the invention is formed by linking at least one thioredoxin and at least one human serum albumin.
Here, thioredoxins are proteins with a molecular weight of 12 kD and Cys-Gly-Pro-Cys as the active site, where those in which a disulfide bond is formed with the two cysteines at the active site are referred to as the oxidized form, and those in which dithiols are formed are referred to as the reduced form. Thioredoxins have the function of reducing disulfide bonds in substrate proteins by being converted from the reduced form to the oxidized form.

The 104-amino acid sequence in SEQ. ID. No.1 is an example of a primary amino acid sequence of thioredoxin. However, the thioredoxins in the present invention may have one or more amino acid deletions, substitutions, and/or additions in their amino acid sequences, provided that they have Cys-Gly-Pro-Cys as the active site and are converted from the reduced form to the oxidized form to reduce disulfide bonds in substrate proteins.
In the present invention, the number of amino acid substitutions, deletions, insertions, and/or additions may range from 1 to about 10, for example.
Thioredoxins may also be proteins encoded by the base sequence shown in SEQ. ID. No. 4. The proteins may also be encoded by base sequences hybridized under stringent conditions with DNA comprising of a sequence that is complementary with part of the base sequence in SEQ. ID No. 4.

Human serum albumin refers to proteins widely distributed in the body, including blood and interstitial fluid, the primary function of which is to maintain normal osmotic pressure in the blood stream and to maintain fluid levels in blood. Not only human serum albumin, but the serum albumin of mammals other than humans may be used (such as monkeys, cows, sheep, goats, horses, swine, rabbits, dogs, cats, mice, and rats). Human serum albumin has been used, for example, in treatments where fluids from blood vessels may be lost, such as surgery, shock, burns, and hypoproteinemia which can result in edema.
The 585-amino acid sequence shown in SEQ. ID. No. 2 may be an example of a primary amino acid sequence of human serum albumin. However, the human serum albumin in the present invention may have one or more amino acid deletions, substitutions, and/ or additions in their amino acid sequences, provided that they maintain normal osmotic pressure in the blood stream and maintain the fluid levels in blood.

The human serum albumin may also be a protein encoded by the base sequence in SEQ. ID. No. 5. The proteins may also be encoded by base sequences hybridized under stringent conditions with DNA comprising of a sequence that is complementary with part of the base sequence in SEQ. ID No. 5.

Some of the amino acids may be chemically modified, such as esterification or amidation of the C terminal, and protection of OH, COOH, NH₂, SH groups and the like included in the amino acids using a suitable protective groups such as a formyl groups.

The thioredoxin and human serum albumin in the fusion protein of the invention may be linked in any way, provided that the function and secondary structure of the each protein is not affected. Specifically, the human serum albumin may be linked to the thioredoxin either directly, or via a linker or spacer, etc., or a carrier protein, tagged polypeptide, or the like is linked to the proteins of the invention, a linker, or a spacer, etc.

Here, the linker or spacer can be composed of one or more amino acids (referred to below as "linker polypeptide" in the Specification) and are not particularly limited, provided that the functions and secondary structure of the each protein is not affected, as noted above. Examples include glycine, methionine, and serine, which are small residues, and those based on glycine are preferred. The linker polypeptide may have 1 to about 30 amino acids, and preferably about 7 to about 15.

The base sequence encoding the amino acid sequence of the linker polypeptide preferably include restriction enzyme cleavage sites to facilitate ligation, that is, the production of a vector for producing the fusion protein of the invention. For example, the sticky terminals of the restriction enzyme cleavage sites can be added to each of a 3' terminal of the base sequence coding for the thioredoxin and a 5' terminal of the base sequence coding for the human serum albumin, and the sticky terminals can be joined by ligation to link the genes encoding them. In such cases, ligation can be accomplished with relative ease because the strand lengths of the genes are not very long despite the addition of two genes to handle and the addition of one more site for ligation. The base sequences encoding the amino acid sequence of the linker polypeptide is formed by the sticky terminals joined.
Specifically, when the restriction enzyme is Aval, the amino acid sequence of the linker polypeptide may be Leu-Gly, and the base sequence coding for the amino acid sequence (which is also the base sequence for the AvaI restriction enzyme cleavage site) will be CCCGAG, CCCGGG, CTCGAG or CTCGGG.

Examples of such a restriction enzyme cleavage site include Accl, AfaI, ApaI, AvaI, AvaII, Ball, BamHI, BbeI, BcnI, BglI, BlnI, ClaI, CpoI, DraI, EaeI, EcoRI, EcoRV, FbaI, FokI, FseI, HaeI, HaeII, HaeIII, HapII, HhaI, HinfI, HpaI, KpnI, MboI, MboII, MluI, MspI, NaeI, NcoI, NotI, SacI, SacII, SalI, ScaI, SmaI, SpeI, StuI, TaqI, XbaI, XhoI, and the like. Also, since the restriction enzyme cleavage sites directly code for part of the linker polypeptide, the base sequence coding for the linker polypeptide can be designed by adding the desired number of bases to the 5' terminal and/or 3' terminal sides of the restriction enzyme cleavage site. As noted above, the design preferably include more glycine, methionine, and serine, particularly glycine.
Specifically, when the restriction enzyme is AvaI, CTCGGG can be selected as the base sequence, GG can be added to the 5' terminal, and an appropriate base can be added to the 3' terminal to produce a base sequence coding for Gly-Ser-Gly.

The amino acid sequence in SEQ. ID. No. 3 is thus an example of a preferred embodiment of the fusion protein of the invention. In this amino acid sequence, the amino acid sequence for the thioredoxin (such as the amino acid sequence in SEQ. ID. No. 1) is a sequence in which the amino acid sequence for the human serum albumin (such as the amino acid sequence in SEQ. ID. No. 2) is linked via the amino acid sequence for a linker polypeptide ([Gly-Gly-Gly-Gly-Ser]₂) encoded by a base sequence that includes the restriction enzyme AvaI.
The protein comprising of the amino acid sequence in SEQ. ID. No. 3 may have one or more amino acid deletions, substitutions, and/ or additions to the amino acid sequence, provided that the functions of the protein with the amino acid sequence in SEQ. ID. No. 3 are substantially uncompromised. Furthermore, a polypeptide corresponding to any one of (1) through (3) below, for example, may be added to either terminal of the amino acid sequence for the fusion protein of the invention.

(1) Restriction enzyme cleavage site- (or sticky terminal)-encoded polypeptides used to produce vectors in the recombinant DNA techniques described below;
(2) polypeptides encoded by a base sequence for initiating and a base sequence for terminating protein synthesis previously provided in a vector, for producing proteins in the recombinant DNA techniques described below; and
(3) polypeptides used to isolate and purify protein products in the recombinant DNA techniques described below. Examples include polypeptides with an amino acid sequence capable of binding to columns.

Any carrier protein will known in the field can be used as a carrier protein. Examples include Keyhole Limpet Hemocyanin (KLH), bovine serum albumin (BSA), ovoalbumin (OVA), and the like. Examples of tagged polypeptides include glutathione-S-transferase (GST), super oxide dismutase (SOD), β-galactosidase, and the like.
The thioredoxin may also be linked to either the N terminal or C terminal side, or both the N terminal and C terminal side of the amino acid sequence for the human serum albumin.

Base sequences that can be hybridized under stringent conditions with the base sequence represented in SEQ. ID. No 4 or 5 described above mean, for example, DNA obtained by a well known method such as colony hybridization or plaque hybridization using the base sequence in SEQ. ID. No. 4 or 5 as probe. "Stringent conditions" mean conditions under which so-called specific hybrids are formed and no non-specific hybrids are formed, such as conditions resulting in the hybridization of DNA of high homology, which is DNA with at least 50%, and preferably at least 60%, at least 80%, or at least 90% homology with the base sequence in SEQ. ID. No. 4 or 5. Specifically, this refers to hybridization conditions at about 60 to 70°C with 0.1x to 2x SSC solution (composition of Ix SSC solution: 150 mM sodium chloride, 15 mM sodium citrate).

The fusion protein of the invention can be produced by chemical synthesis or recombinant DNA techniques that are well known per se. For example, DNA having a base sequence encoding the amino acid sequence of the fusion protein of the invention can be inserted into a vector, the host cells can be transformed in the vector, the transformants can be cultured under conditions suitable for the expression of the fusion protein, the protein can be expressed, and the resulting fusion protein can be recovered from the cells or culture media. However, because shorter gene strand lengths are preferred in the interests of facilitating ligation during incorporation into the vector, an appropriately divided gene may be introduced into the vector, either simultaneously or in stages. The gene can be introduced into the vector by well known means. Specifically, specific restriction enzyme cleavage sties in the vector are preferably cleaved with specific restriction enzymes, and the gene of the invention is preferably inserted at those cleavage sites.

It is essential to select the restriction enzymes and/or to design a base sequence that codes for thioredoxin and human serum albumin in order to avoid accidentally digesting the base sequences coding for thioredoxin and human serum albumin during treatment with the restriction enzymes. For example, a gene having a base sequence coding for the thioredoxin of SEQ. ID. No. 4 and the base sequence coding for the human serum albumin of SEQ. ID. No. 5 will not be digested by the restriction enzymes AvaI, XhoI, and EcoRI, and may therefore be treated with those restriction enzymes.

Any vector well known in the field can be used and may be selected upon a consideration of the appropriate combination with the intterminaled host cell. The vector is not particularly limited, provided that it is a viral vector, plasmid, phage, cosmid, or the like capable of autonomous replication in the cell to which it is introduced. Suitable selection markers (such as drug resistance genes) and/ or promoters, as well as other types of expression-regulating elements (such as terminators and enhancers) may also be included. For the sake of easier manipulation, the vector is preferably a plasmid, for example, which has been constructed as a recombinant vector. The host cells are not particularly limited, provided that they permit stable autonomous vector replication and stable expression of exogenous gene traits. Examples include prokaryotic cells such as *Escherichia coli,* and eukaryotic cells such as yeast (e.g. *Saccharomyces cerevisiae*), insect, plant cells and animal cells. Examples of well known methods for the introduction of vectors into host cells include conjugation, electroporation, competent cell methods, and the like.
The appropriate conditions for the expression of the fusion protein of the invention (such as culture media and culture temperature) can be determined as needed upon a consideration of the host cells, the vectors used for expression, and the like.
The fusion protein of the invention can also be chemically synthesized by a method known in the field, such as Merrifield solid phase synthesis and methods employing commercially available polypeptide synthesizers.

The above fusion protein of the invention can be used as a medicinal composition, for example, that includes the fusion protein of the invention as an active ingredient. For example, the fusion protein of the invention can be directly administered to mammals, including humans, to induce or control immune response and bring about anti-inflammatory action in humans or the like in a stable manner over a long period of time.
In such cases, the fusion protein of the invention can be used without modification, but usually pharmaceutically and pharmacologically acceptable additives, excipients, and the like are preferably used to produce and use a medicinal composition containing the fusion protein of the invention as an active ingredient.

The route by which the medicinal composition is administered is not particularly limited. Examples include any route of administration such as intradermal, subcutaneous, intramuscular, intraperitoneal, transdermal, transmucosal, oral, inhalation administration, and the like, although parenteral routes of administration, especially administration by injection, are preferred.
The dosage form of the medicinal composition of the invention can be any that is suitable for administration by the above routes of administration, such as injections, patches, cataplasms, ophthalmic solutions, nasal solutions, sprays, tablets, capsules, lozenges, sublingual tablets, creams, lotions, and powders. It may also be encapsulated in biodegradable liposomes, microcapsules, or microspheres, and may also be formulated in lyophilized form.

Particularly in the case of injections, the fusion protein may be in the form of a solution or suspension. Additives commonly used in the field, such as pH regulators, electrolytes, saccharides, vitamins, pharmacologically acceptable salts or fatty acids and/or amino acids, may be added as needed.
For solutions and suspensions, any solution stipulated in the Japanese Pharmacopoeia, for example, can be used. Water (water for injection), normal saline, and various types of buffers (such as phosphate buffer) can primarily be used.

pH regulators commonly used in injection should be used. Examples include organic acids such as citric acid, tartaric acid, acetic acid, and lactic acid; inorganic acids such as hydrochloric acid and phosphoric acid; and inorganic bases such as sodium bicarbonate, sodium carbonate, and sodium hydroxide.

A variety of water-soluble salts conventionally used in infusions can be used as the electrolytes. Examples include various inorganic (for example, sodium, potassium, calcium, magnesium, phosphorus an eth elike) water-soluble salts (for example, chloride, sulfate, acetate, gluconate, lactate and the like) that are considered necessary in terms of maintaining bodily functions or the humoral electrolyte balance.

A variety of saccharides conventionally used in infusions can be used. Glucose is an example. Examples include monosaccharides such as glucose, fructose, galactose; disaccharides such as lactose, maltose; polyols such as glycerol; sugar alcohols such as xylitol, sorbitol, mannitol; Dexetrines such as Dexetrine 40 or Dexetrine 80; sucrose, and the like.

Various conventionally used water-soluble/liposoluble vitamins can be used. Examples include Vitamin A, provitamin A, vitamin D, provitamin D, vitamin E, vitamin K, vitamin B1, vitamin B2, niacin, vitamin B6 groups, pantothenic acid, biotin, Mio-inositol, Colin, folic acid, vitamin B12, vitamin C, vitamin P, vitamins U, and the like.

Examples of pharmacologically acceptable salts or fatty acids include alkali metals such as sodium, potassium; alkaline-earth metals such as calcium and magnesium; organic acid such as formic acid, acetic acid, oxalic acid, tartaric acid, maleic acid, citric acid, caprylic acid, succinic acid and malic acid; organic bases such as trimethylamine, triethylamine, pyridine, picoline, ethanolamine, diethanolamine, triethanolamine and dicyclohexylamine.

Examples of amino acids include, for example, alanine, arginine, asparagine, aspartic acid, cysteine, glutamine, glutaminic acid, glycine, histidine, hydroxyproline, isoleucine, leucin, lysin, methionine, phenylalanine, proline, serine, threonine, tryptophan, tylosin and valine. In addition, amino acid derivatives such as N - acetyl methionine may also be used.

Injections can be manufactured in accordance with the usual pharmaceutical procedures. That is, a medicinal composition containing the fusion protein of the invention can be prepared through dilution and/or dissolution to a final fusion protein concentration of between about 10 mg/mL and about 100 mg/mL, and a pH of about 4.5 to about 8.7 using various types of buffers, common commercially available infusions (such as amino acid infusions and electrolyte infusions) or aqueous solutions containing ingredients similar to these, and the like.
The fused protein per unit is not particularly limited, but may be, for example, prepared in the form of an injection containing about 1 to about 1000 mg, and preferably about 10 to about 100 mg.

The following are examples of the invention, but the invention is not limited to these examples.

### Example 1

The fusion protein of the invention was produced in the following manner.

### (1) Preparation of Thioredoxin DNA Fragments

A thioredoxin gene DNA library was produced by the extraction and reverse transcription of mRNA from U937 cells. PCR of the DNA library was carried out with a polymerase (KOD-plus-Ver. 2, by Toyobo co. Ltd.) using a sense primer of SEQ. ID. No. 6 and an antisense primer of SEQ. ID. No. 7 as synthesis primers. The PCR conditions comprised 2 minutes of DNA treatment at 94°C followed by 30 cycles of denaturation (94°C, 15 sec), annealing (65°C, 30 sec), and extension (68°C, 1 min). The PCR gave DNA fragments comprising AvaI added to the 5' terminal and EcoRI added to the 3' terminal of the base sequence (SEQ. ID. No. 4) coding for thioredoxin.
The primer
   SEQ. ID. No. 6 : ggtaccctcg agaaaagaga tgcacacaag agtgaggttg c 41
The antisense primer
   SEQ. ID. No. 7: cagctgcccg agccaccacc acctaagcct aaggcagctt gacttgc 47

### (2) Preparation of Human Serum Albumin DNA Fragments

PCR was carried out with a polymerase (KOD-plus-Ver. 2, by Toyobo) using as template a plasmid obtained by incorporating the human serum albumin gene into the pPIC9 plasmid (hereinafter " pPIC9-HAS") and using as synthesis primers a sense primer of SEQ. ID. No. 8 and an antisense primer of SEQ. ID. No. 9. The PCR conditions comprised 2 minutes of DNA treatment at 94°C followed by 30 cycles of denaturation (94°C, 15 sec), annealing (65°C, 30 sec), and extension (68°C, 1 min). The PCR gave DNA fragments comprising XhoI added to the 5' terminal and AvaI added to the 3' terminal of the base sequence (SEQ. ID. No. 5) coding for human serum albumin.
The primer
   SEQ. ID. No. 8 : gttaacctcg ggcggtggcg gaagtgtgaa gcagatcgag agcaag 46
The antisense primer
   SEQ. ID. No. 9 : gagagaattc gaattccaag tttaaatagc caatggctgg 40

### (3) Preparation of Plasmid

The human serum albumin and thioredoxin DNA fragments amplified by PCR were extracted with phenol and then purified by ethanol precipitation. The human serum albumin DNA fragments were digested with the restriction enzymes Xhol (by Takara Shuzo) and AvaI (by Toyobo), and the thioredoxin DNA fragments were digested with AvaI and Eco RI (by Toyobo). Following treatment with the restriction enzymes, the DNA fragments were electrophoresed on agarose, and the bands corresponding to the DNA fragments (DNA fragments encoding albumin: HAS; DNA fragments encoding thioredoxin: Trx) were cut out for gel extraction using a gel extraction kit (QIAquick Gel Extraction Kit, by QIAGEN). The DNA fragments obtained by gel extraction (HAS, Trx and pPIC9) were ligated for 30 min at 16°C using a DNA ligation kit (DNA Ligation Kit, Ver. 2.1, by Takara Shuzo) so as to prepare a recombinant plasmid comprising the DNA fragments coding for albumin and thioredoxin joined to the plasmid pPIC9 (pPIC9-HAS-Trx).

### (4) Plasmid Verification

The pPIC9-HSA-Trx obtained in section (3) above was introduced into *E*. *coli* DH5α (by Takara Shuzo) for transformation. The transformants incorporating the pPIC9-HSA-Trxwere screened in ampicillin media, and plasmids were purified from the resulting transformants (QIAprep Spin Miniprep Kit, by QIAGEN). A restriction map was prepared by plasmid digestion with BglII (Takara Shuzo), double digestion with BamHI and SalI (Toyobo), and double digestion with AvaI and EcoRI (Toyobo). The plasmids obtained above were thus confirmed to be the target plasmid.

### (5) Verification of Base Sequences in Plasmid

The base sequences coding for amino acids including the human serum albumin and thioredoxin in the pPIC9-HSA-Trx obtained in section (3) of Example 1 were checked with an ABI Prism 310 Genetic Analyzer (Applied Biosystems) using a α-factor sequencing primer of SEQ. ID. No. 10 (by Invitrogen) and sequencing primers of SEQ. ID. Nos. 11 through 17.
The pPIC9-HSA-Trx was thus confirmed to have the base sequences of SEQ. ID. Nos. 4 and 5.
The α-factor sequencing primer
   SEQ. ID. No. 10 : tactattgcc agcattgctg c 21
The sequencing primers
   SEQ. ID. No.11 : cctatggtga aatggctgac tgctgtgc 27
   SEQ. ID. No. 12: gccagaagac atccttactt ttatgccccg c 31
   SEQ. ID. No. 13: gacagggcgg accttgccaa gtatatctg 29
   SEQ. ID. No. 14: cccactgcat tgccgaagtg gaaaatgatg 30
   SEQ. ID. No. 15: gttcgttaca ccaagaaagt accccaagtg 30
   SEQ. ID. No. 16: catgcagata tatgcacact ttctgag 27
   SEQ. ID. No. 17 : gcagatcgag agcaagactg cttttcagga agcc 34

### (6) Preparation of Fusion Protein

Digestion of the pPIC9-HSA-Trx with the restriction enzyme SalI was followed by phenol extraction and then purification by ethanol precipitation. Transformation was then brought about by homologous transformation to the HIS4 gene locus of *Pichia pastoris* (GS115 strain) using an electroporation device (Gene Pulser 11 Electroporation System, by BIO-RAD). The transformed *Pichia pastoris* was incubated for 48 hours in BMGY liquid media, and was then cultured for 96 hours as 1 % methanol was added every 24 hours to the BMMY media. The yeast was then isolated by centrifugation (6,000 G, 10 min), ammonium sulfate was then added to 30% (v/v) in the broth, and the impurities were centrifuged off. Ammonium sulfate was then added to 80% (v/v) in the supernatant to adjust the pH to 5.5, and the contents were stirred over night. The target protein was then precipitated by centrifugation (15,000 G, 20 min). The precipitated protein was dissolved in 20 mM Tris/HCl (pH 8.0), dialyzed, and then allowed to bind to a HiTrap Q XL column (by GE Healthcare), and the fusion protein was eluted with a 0 → 500 mM NaCl concentration gradient. The eluate was thterminalialyzed against 1.5 M ammonium sulfate/50 mM Tris/HCl (pH 7.0) and allowed to flow through a HiTrap Phenyl HP column (by GE Healthcare), and the fusion protein was eluted with a 1.5 → 0 M sulfuric acid gradient. The fusion protein of the invention was obtained by delipidation with activated carbon.

### Comparative Example 1: Recombinant Human Serum Albumin

Recombinant human serum albumin (provided by Baifa) was used for comparison with the fusion protein of the invention in Text Examples 1 through 5 below.

### Comparative Example 2: Native Human Serum Albumin

Native human serum albumin (provided by Kaketsukenn) was used for comparison with the fusion protein of the invention in Text Examples 1 through 5 below.

### Comparative Example 3: Thioredoxin

Thioredoxin (provided by R&D system) was used for comparison with the fusion protein of the invention in Text Examples 2, 3 through 5 below.

### Comparative Example 4: Phosphate buffer

Phosphate buffer (PBS) was used for comparison with the fusion protein of the invention in Text Example 4 below.

### Test Example 1: Verification of Fusion Protein by Gel Electrophoresis

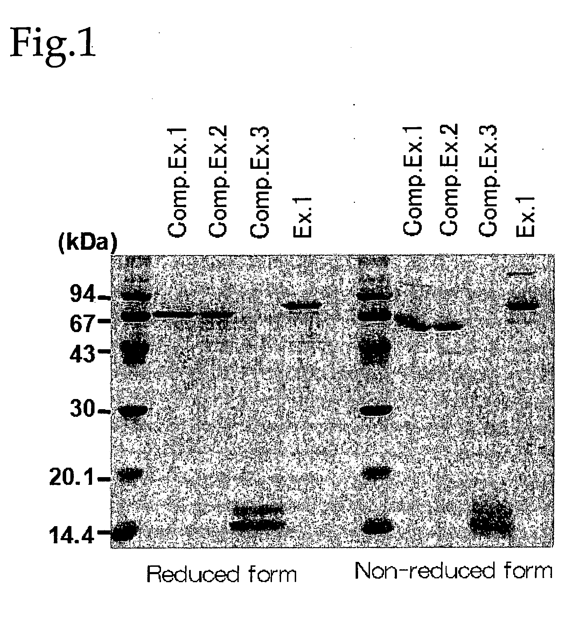

The fusion protein produced above was electrophoresed on 12.5% SDS-polyacrylamide gel. Recombinant human serum albumin (Comparative Example 1), native human serum albumin (Comparative Example 2), and thioredoxin (Comparative Example 3) were used as controls. Measurements were done on samples that had and had not been reduced with the addition of 1,4-dithiothreitol (DTT). The results are given in Figure 1.
The results in Figure 1 suggest that the thioredoxin was linked to the human serum albumin in the fusion protein because bands were found more on the high molecular weight side compared to the recombinant human serum albumin (Comparative Example 1) and the natural human serum albumin (Comparative Example 2).

### Test Example 2: Verification of Fusion Protein by Western Blotting

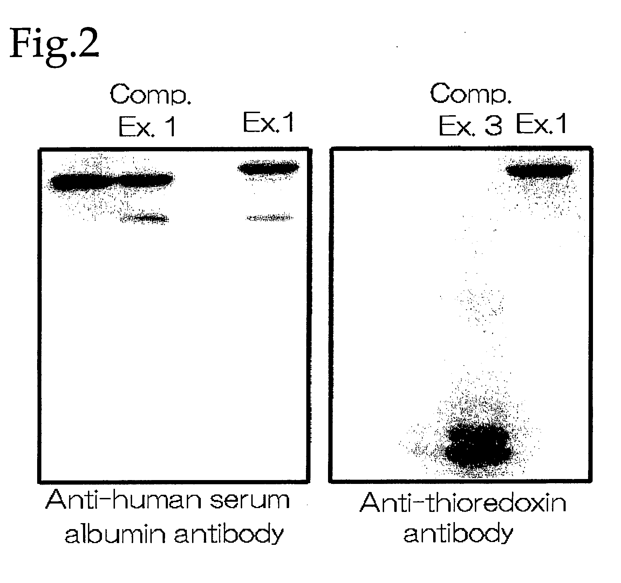

The fusion protein produced above was analyzed by Western blotting using anti-human serum albumin antibody and anti-thioredoxin antibody. Human serum albumin (Comparative Example 1) and thioredoxin (Comparative Example 3) were used as controls. The results are given in Figure 2.
According to Figure 2, the anti-human serum albumin antibodies resulted in bands for the recombinant human serum albumin (Comparative Example 1) and the fusion protein. Western blotting with anti-thioredoxin antibody revealed bands for thioredoxin (Comparative Example 3) and the fusion protein. This suggested that thioredoxin was linked to the human serum albumin in the fusion protein.

### Test Example 3: Verification of Fusion Protein by ELISA

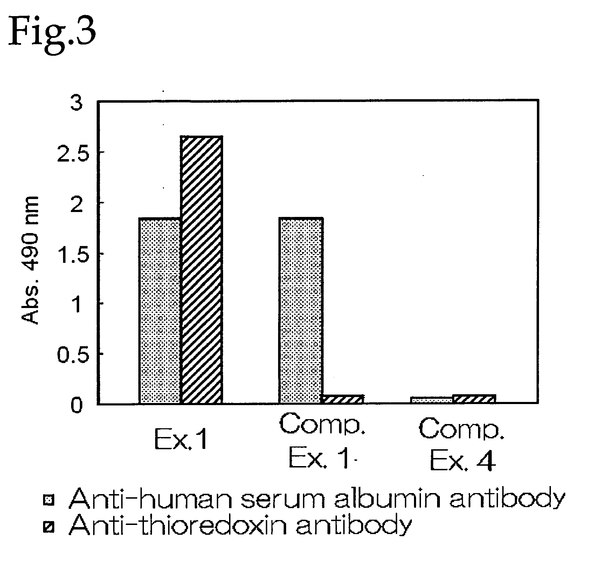

ELISA was run with anti-human serum albumin antibody and anti-thioredoxin antibody. Recombinant human serum albumin (Comparative Example 1) was used as the control. The results are given in Figure 3.
According to Figure 3, the reactivity to the recombinant human serum albumin (Comparative Example 1) and the fusion protein was about the same. On the other hand, the anti-thioredoxin antibody did not bind at all to the human serum albumin, but clearly reacted very much with the fusion protein.
This suggested that thioredoxin was linked to the human serum albumin in the fusion protein.

### Test Example 4: Verification of Thioredoxin Activity in Fusion Protein

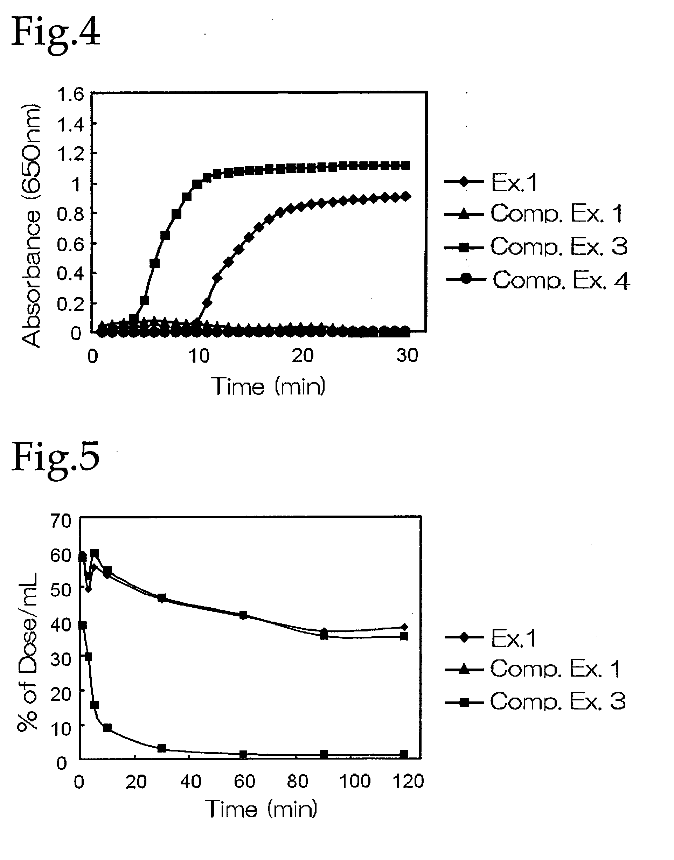

The thioredoxin activity of the fusion protein was verified. Specifically, insulin was dissolved to 5 mg/mL in a calcium phosphate (pH 7.0) solution of the fusion protein, DTT was further added to 5 mM so as to reduce the insulin, and the precipitated insulin was evaluated based on the changes over time in the optical density at 650 nm. Recombinant human serum albumin (Comparative Example 1), thioredoxin (Comparative Example 3), and PBS (Comparative Example 4) were used as control. The results are given in Figure 4.
According to Figure 4, the thioredoxin (Comparative Example 3) caused the insulin to precipitate after 4 min, and reached a plateau after 10 min. The fusion protein resulted in precipitation after 10 min and reached a plateau after 19 min. On the other hand, the recombinant human serum albumin (Comparative Example 1) and the PBS (Comparative Example 4) did not result in the precipitation of insulin.
It was thus clear that the fusion protein had sufficient thioredoxin activity.

### Test Example 5: Verification of Retention of Fusion Protein in Blood

The retention of the fusion protein in blood was verified. Specifically, the lysine residue of the fusion protein was first labeled with a radioactive indium isotope (111In). The fusion protein was then administered via the caudal vein to mice (dose: 0.1 mg/kg), and the plasma concentration profile was determined with a radiometric device. The results are given in Figure 5.
According to Figure 5, the elimination of the fusion protein in blood was about the same as that of the recombinant albumin (Comparative Example 1), but the thioredoxin (Comparative Example 3) was rapidly eliminated from blood. It was thus clear that producing the fusion protein with albumin dramatically improved the retention of thioredoxin in blood.

As described above, the fusion protein of the invention is a fusion protein of thioredoxin and human serum albumin, which allows the activity of thioredoxin to be maintained over a long period of time. It is thus possible to provide a protein preparation that is stable over long periods of time. The vector and transformants of the invention also allow the fusion protein to be readily produced by recombinant techniques.

This application claims priority to Japanese Patent Application No. 2007-225833. The entire disclosure of Japanese Patent Application No. 2007-225833 is hereby incorporated herein by reference.
While only selected embodiments have been chosen to illustrate the present invention, it will be apparent to those skilled in the art from this disclosure that various changes and modifications can be made herein without departing from the scope of the invention as defined in the appterminaled claims. Furthermore, the foregoing descriptions of the embodiments according to the present invention are provided for illustration only, and not for the purpose of limiting the invention as defined by the appterminaled claims and their equivalents. Thus, the scope of the invention is not limited to the disclosed embodiments.

## Claims

1. Fusion protein comprising a thioredoxin and human serum albumin.

2. Fusion protein according to Claim 1, wherein the thioredoxin comprises
(a) a protein comprising the amino acid sequence shown in SEQ. ID. No.1, or
(b) a protein comprising the amino acid sequence in SEQ. ID. No.1 with one or more amino acid substitutions, deletions, insertions, and/ or additions, that is functionally equivalent to the protein comprising the amino acid sequence in SEQ. ID. No.1.

3. Fusion protein according to Claim 1, wherein the human serum albumin comprises
(a) a protein comprising the amino acid sequence shown in SEQ. ID. No. 2, or
(b) a protein comprising the amino acid sequence in SEQ. ID. No. 2 with one or more amino acid substitutions, deletions, insertions, and/ or additions, that is functionally equivalent to the protein comprising the amino acid sequence in SEQ. ID. No. 2.

4. Fusion protein according to Claim 1, wherein the fusion protein comprises
(a) a protein comprising the amino acid sequence shown in SEQ. ID. No. 3, or
(b) a protein comprising the amino acid sequence in SEQ. ID. No. 3 with one or more amino acid substitutions, deletions, insertions, and/ or additions, that is functionally equivalent to the protein comprising the amino acid sequence in SEQ. ID. No. 3.

5. Fusion protein according to Claim 1, wherein the thioredoxin encoded by
(a) a base sequence shown in SEQ. ID. No. 4, or
(b) a base sequence hybridized under stringent conditions with DNA comprising of a sequence that is complementary with part of the base sequence
in SEQ. ID No. 4, that encodes a protein which is functionally equivalent to the protein encoded by a DNA comprising the base sequence in SEQ. ID. No. 4.

6. Fusion protein according to Claim 1, wherein the human serum albumin encoded by
(a) a base sequence shown in SEQ. ID. No. 5, or
(b) a base sequence hybridized under stringent conditions with DNA comprising of a sequence that is complementary with part of the base sequence in SEQ. ID No. 5, that encodes a protein which is functionally equivalent to the protein encoded by a DNA comprising the base sequence in SEQ. ID. No. 5.

7. Gene encoding
(a) a protein comprising the amino acid sequence shown in SEQ. ID. No. 3, or
(b) a protein comprising the amino acid sequence in SEQ. ID. No. 3 with one or more amino acid substitutions, deletions, insertions, and/ or additions, that encodes a protein which is functionally equivalent to the protein comprising the amino acid sequence in SEQ. ID. No. 3.

8. A vector comprising the gene according to Claim 7.

9. Transformant comprising the vector according to Claim 8.

10. Fusion protein produced by the transformants according to Claim 9.

11. An anti-inflammatory medicinal composition comprising the fusion protein according to Claim 1.


SEQUENCE LISTING

<110>  Nipro Corporation

<120>  Fusion Protein of Thioredoxine and Albumin

<130>  JP070926P

<160>  17

<170>  PatentIn version 3.1

<210>  1
<211>  104
<212>  Protain
<213>  Thioredoxine

<400>  1
Val Lys Gln Ile Glu Ser Lys Thr Ala Pro Gln Glu Ala Leu Asp Ala
1               5                   10                  15

Ala Gly Asp Lys Leu Val Val Val Asp Pro Ser Ala Thr Trp Cys Gly
            20                  25                  30

Pro Cys Lys Met Ile Lys Pro Pro Pro His Ser Leu Ser Glu Lys Tyr
        35                  40                  45

Ser Asn Val Ile Pro Leu Glu Val Asp Val Asp Asp Cys Gln Asp Val
    50                  55                  60

Ala Ser Glu Cys Glu Val Lys Cys Met Pro Thr Pro Gln Pro Pro Lys
65                  70                  75                  80

Lys Gly Gln Lys Val Gly Glu Pro Ser Gly Ala Asn Lys Glu Lys Leu
                85                  90                  95

Glu Ala Thr Val Asn Glu Leu Val
            100

<210>  2
<211>  585
<212>  Protain
<213>  Albumin

<400>  2


<210>  3
<211>  699
<212>  Protain
<213>  Fusion Protain

<400>  3


<210>  4
<211>  312
<212>  DNA
<213>  Artificial

<220>
<223>  Designed DNA based on Thioredoxine

<400>  4
gtgaagcaga tcgagagcaa gactgctttt caggaagcct tggacgctgc aggtgataaa   60

cttgtagtag ttgacttctc agccacgtgg tgtgggcctt gcaaaatgat caagcctttc  120

tttcattccc tctctgaaaa gtattccaac gtgatattcc ttgaagtaga tgtggatgac  180

tgtcaggatg ttgcttcaga gtgtgaagtc aaatgcatgc caacattcca gttttttaag  240

aagggacaaa aggtgggtga attttctgga gccaataagg aaaagcttga agccaccatt  300

aatgaattag tc                                                      312


<210>  5
<211>  1755
<212>  DNA
<213>  Artificial

<220>
<223>  Designed DNA based on Albumin

<400>  5
gatgcacaca agagtgaggt tgctcatcgg tttaaagatt tgggagaaga aaatttcaaa   60

gccttggtgt tgattgcctt tgctcagtat cttcagcagt gtccatttga agatcatgta  120

aaattagtga atgaagtaac tgaatttgca aaaacatgtg ttgctgatga gtcagctgaa  180

aattgtgaca aatcacttca tacccttttt ggagacaaat tatgcacagt tgcaactctt  240

cgtgaaacct atggtgaaat ggctgactgc tgtgcaaaac aagaacctga gagaaatgaa  300

tgcttcttgc aacacaaaga tgacaatcca aatctccccc gattggtgag accagaggtt 360
gatgtgatgt gcactgcttt tcatgacaat gaagagacat ttttgaaaaa atacttatat 420
gaaattgcca gaagacatcc ttacttttat gccccgcaac tccttttctt tgctaaaagg 480
tataaagctg cttttacaga atgttgccaa gctgctgata aagcagcctg cctgttgcca 540
aagctcgatg aacttcggga tgaagggaag gcttcgtctg ccaaacagag actcaagtgt 600
gccagtctcc aaaaatttgg agaaagagct ttcaaagcat gggcagtagc tcgcctgagc 660
cagagatttc ccaaagctga gtttgcagaa gtttccaagt tagtgacaga tcttaccaaa 720
gtccacacgg aatgctgcca tggagatctg cttgaatgtg ctgatgacag ggcggacctt 780
gccaagtata tctgtgaaaa tcaagattcg atctccagta aactgaagga atgctgtgaa 840
aaacctctgt tggaaaaatc ccactgcatt gccgaagtgg aaaatgatga gatgcctgct 900
gacttgcctt cattagcggc tgattttgtt gaaagtaagg atgtttgcaa aaactatgct 960
gaggcaaagg atgtcttctt gggcatgttt ttgtatgaat atgcaagaag gcatcctgat 1020
tactctgtcg tactgctgct gagacttgcc aagacatatg aaaccactct agagaagtgc 1080
tgtgccgctc cagatcctca tgaatgctat gccaaagtgt tcgatgaatt taaacctctt 1140
atggaagagc ctcagaattt aatcaaacaa aattgtgagc tttttgagca gcttggagag 1200
tacaaattcc agaatgcgct attagttcgt tacaccaaga aagtacccca agtgtcaact 1260
ccaactcttg tagaggtctc aagaaaccta ggaaaagtgg gcagcaaatg ttgtaaacat 1320
cctgaagcaa aaagaatgcc ctgtgcagaa gactatctat ccgtggtcct gaaccagtta 1380
tgtgtgttgc atgagaaaac gccagtaagt gacagagtca ccaaatgctg cacagaatcc 1440
ttggtgaaca ggcgaccatg cttttcagct ctggaagtcg atgaaacata cgttcccaaa 1500
gagtttaatg ctgaaacatt caccttccat gcagatatat gcacactttc tgagaaggag 1560
agacaaatca agaaacaaac tgcacttgtt gagcttgtga aacacaagcc caaggcaaca 1620
aaagagcaac tgaaagctgt tatggatgat ttcgcagctt ttgtagagaa gtgctgcaag 1680
gctgacgata aggaaacctg ctttgccgag gagggtaaaa aacttgttgc tgcaagtcaa 1740
gctgccttag gctta 1755

<210> 6
<211> 41
<212> DNA
<213> Artificial

<220>
<223> Designed DNA based on Thioredoxine (primer)

<400> 6
ggtaccctcg agaaaagaga tgcacacaag agtgaggttg c 41

<210> 7
<211> 47
<212> DNA
<213> Artificial

<220>
<223>  Designed DNA based on Thioredoxine (primer)

<400>  7
cagctgcccg agccaccacc acctaagcct aaggcagctt gacttgc                    47


<210>  8
<211>  46
<212>  DNA
<213>  Artificial

<220>
<223>  Designed DNA based on Albumin (primer)

<400>  8
gttaacctcg ggcggtggcg gaagtgtgaa gcagatcgag agcaag                    46


<210>  9
<211>  40
<212>  DNA
<213>  Artificial

<220>
<223>  Designed DNA based on Albumin (primer)

<400>  9
gagagaattc gaattccaag tttaaatagc caatggctgg                    40


<210>  10
<211>  21
<212>  DNA
<213>  Artificial

<220>
<223>  Designed DNA based on Fusion Protain (primer)

<400>  10
tactattgcc agcattgctg c                    21


<210>  11
<211>  27
<212>  DNA
<213>  Artificial

<220>
<223>  Designed DNA based on Fusion Protain (primer)

<400>  11
cctatggtga aatggctgac tgctgtgc                    27


<210>  12
<211>  31
<212>  DNA
<213>  Artificial

<220>
<223>  Designed DNA based on Fusion Protain (primer)

<400>  12
gccagaagac atccttactt ttatgccccg c                    31


<210>  13

<211>  29
<212>  DNA
<213>  Artificial

<220>
<223>  Designed DNA based on Fusion Protain (primer)

<400>  13
gacagggcgg accttgccaa gtatatctg                                    29


<210>  14
<211>  30
<212>  DNA
<213>  Artificial

<220>
<223>  Designed DNA based on Fusion Protain (primer)

<400>  14
cccactgcat tgccgaagtg gaaaatgatg                                   30


<210>  15
<211>  30
<212>  DNA
<213>  Artificial

<220>
<223>  Designed DNA based on Fusion Protain (primer)

<400>  15
gttcgttaca ccaagaaagt accccaagtg                                   30


<210>  16
<211>  27
<212>  DNA
<213>  Artificial

<220>
<223>  Designed DNA based on Fusion Protain (primer)

<400>  16
catgcagata tatgcacact ttctgag                                      27


<210>  17
<211>  34
<212>  DNA
<213>  Artificial

<220>
<223>  Designed DNA based on Fusion Protain (primer)

<400>  17
gcagatcgag agcaagactg cttttcagga agcc                              34